# EUROPÄISCHE PATENTSCHRIFT

(11) **EP 0 536 524 B1**
(45) Veröffentlichungstag und Bekanntmachung des Hinweises auf die Patenterteilung: **11.10.1995**
(21) Anmeldenummer: 92114597.5
(22) Anmeldetag: 27.08.1992
(51) Int. Cl.: A61B 17/39

(54) **Endoskopische Koagulationsfasszange**
Endoscopic prehension and coagulation forceps
Pince de préhension et de coagulation endoscopique

(30) Priorität: 11.09.1991 DE 4130064
(43) Veröffentlichungstag der Anmeldung: 14.04.1993
(73) Patentinhaber: Richard Wolf GmbH, 75438 Knittlingen (DE)
(72) Erfinder: Boebel, Manfred, W-7136 Oetisheim (DE); Hiltebrandt, Siegfried, W-7134 Knittlingen (DE)
(74) Vertreter: Vollmann, Heiko, Dipl.-Ing.

(56) Entgegenhaltungen:
- DE-A- 2 415 263
- DE-A- 4 032 471
- GB-A- 2 161 082
- US-A- 2 032 860

## Beschreibung

Die Erfindung geht aus von einer bipolaren Faßzange zum Koagulieren von Gewebe mit zwei am distalen Zangenende befindlichen, Elektroden bildenden Maulteilen zum Fassen und Halten des zu koagulierenden Gewebes, wobei an einem Maulteil ein Steg vorgesehen ist, der bei Schließstellung der Maulteile in und ggf. durch das andere Maulteil greift.

Eine derartige Koagulationszange ist z. B. Gegenstand des DE-GM 75 18 245. Diese Zange ist speziell für die Tubensterilisation durch Koagulation des Eileiters vorgesehen und zu diesem Zweck mit Maulteilen versehen, die an dem zu koagulierenden Eileiter angepaßt sind. Dabei ist einer der Maulteile distalseitig hakenförmig umgebogen, um den Eileiter und die Mesosalpinx, für den entsprechenden Behandlungsvorgang in die Bauchhöhle ziehen zu können. Eine solche Verlagerung hat den Vorteil, daß der Koagulationsvorgang in sicherem Abstand zu umliegenden Körperpartien erfolgen kann, so daß eine Schädigung solcher Gewebeteile vermieden wird. Durch die beschriebene Ausbildung wird erreicht, daß die Mesosalpinx nicht nur über den Eileiter, sondern zusätzlich direkt durch die stirnseitige Umbiegung sicher gehalten wird.

Aus der DE-AS 23 25 626 ist weiter eine Zange für die Elektrokoagulation von anatomischen Gebilden mittels Hochfrequenzstrom bekannt. Diese Zange weist zwei Paare von gegenseitig elektrisch isolierten Klemmbacken auf, die an ihren freien Enden als Greifhaken ausgebildet und gegeneinander bewegbar sind.

Bei der Tubensterilisation durch Koagulation kommt es u. a. darauf an, daß die Mesosalpinx bei dem Koagulationsvorgang nicht beschädigt wird, um in Verbindung mit Blutungen und/oder der Vernarbung der beschädigten Mesosalpinx einhergehende Beschwerden und Gefährdungen zu vermeiden bzw. auszuschließen.

In dieser Beziehung weisen die bekannten Ausführungen Mängel auf, die zu beheben Aufgabe der vorliegenden Erfindung ist.

Diese Aufgabe wird bei der eingangs beschriebenen bipolaren Koagulationszange erfindungsgemäß dadurch gelost, daß das eine erste Maulteil am distalen Ende eine Ausnehmung hat und daß am zweiten Maulteil ein Dorn vorgesehen ist, der bei Schließstellung der Maulteile die Ausnehmung mit Spiel durchgreift, wobei der das zu koagulierende Gewebe aufnehmende Bereich zwischen den Maulteilen distal durch den Dorn und proximal durch einen Schenkel des Steges begrenzt ist.

Die damit erzielbaren Vorteile bestehen insbesondere darin, daß die Mesosalpinx unter Nutzung ihrer Elastizität und außerhalb des die Koagulationswirkung entfaltenden Bereichs ohne Beschädigung unabhängig von dem Eileiter formschlüssig erfaßt wird und damit unter Entlastung des Eileiters in die Bauchhöhle gezogen und sicher festgehalten werden kann.

Eine bessere Feldverteilung des HF-Stromes und gleichzeitig günstige Positionierung von schlauchförmigen Gewebestrukturen wie Eileitern in den Wirkbereich der Koagulationszange kann dadurch erreicht werden, daß ein Ende des hakenförmig gekrümmten Steges mit einem ersten Quersteg verbunden ist, der die Ausnehmung am distalen Ende des ersten Maulteiles proximal begrenzt, während das andere Siegende mit einem gegenüberliegenden zweiten Quersteg dieses Maulteils verbunden ist und daß der Steg eine weitere Ausnehmung des ersten Maulteiles überbrückt und in einer Ebene verläuft, die senkrecht auf der Faßebene der Maulteile steht. Dieser Effekt kann noch dadurch optimiert werden, daß die Maulteile auf einem Teil ihrer Länge einen wellenförmigen Verlauf aufweisen.

Dadurch, daß der Steg bei Schließstellung der Maulteile mit dem Scheitel seiner Hakenform in eine Längsausnehmung des zweiten Maulteils greift und daß der Stegschenkel vom Scheitel des Sieges bogenförmig zum ersten Quersteg verläuft, ergibt sich auch quer zur Erstreckung der Maulteile der Koagulationszange eine sichere Fixierung des ergriffenen Gewebes.

Schließlich kann dadurch, daß der am zweiten Maulteil befestigte, an seinem freien Ende atraumatisch gestaltete Dorn aus PTFE besteht, sichergestellt werden, daß das HF-Feld durch diese Struktur keine Störung erfährt.

Die erfindungsgemäße Koagulationszange wird nachstehend anhand der Zeichnungen näher erläutert. Es zeigen:
- Figur 1: eine Seitenansicht der Koagulationszange in geschlossenem Zustand,
- Figur 2: eine Draufsicht auf die Koagulationszange nach Figur 1,
- Figur 3: eine Seitenansicht der Zange in geöffnetem Zustand,
- Figur 4: eine Draufsicht auf die Zange nach Figur 1 in Darstellung mit abgebrochenem oberen Maulteil.

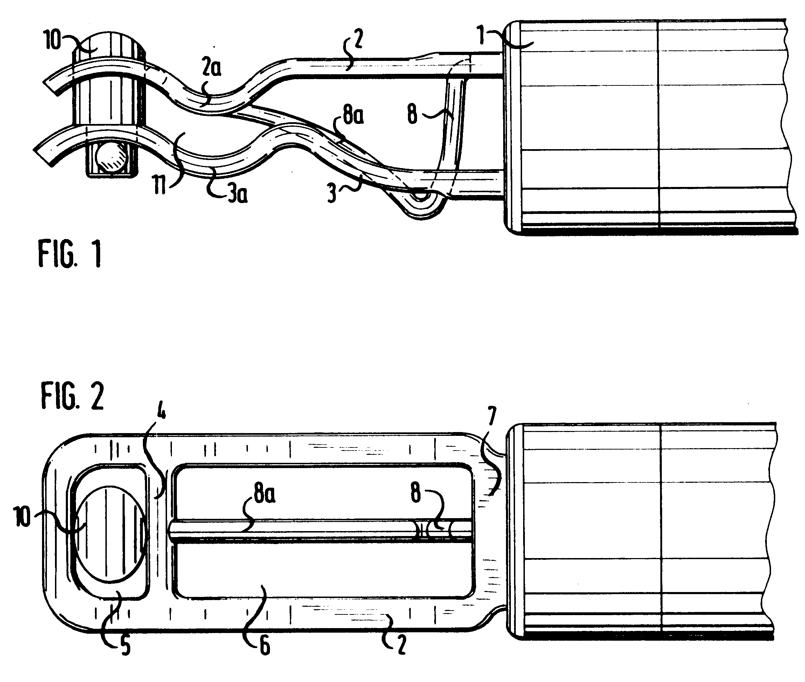

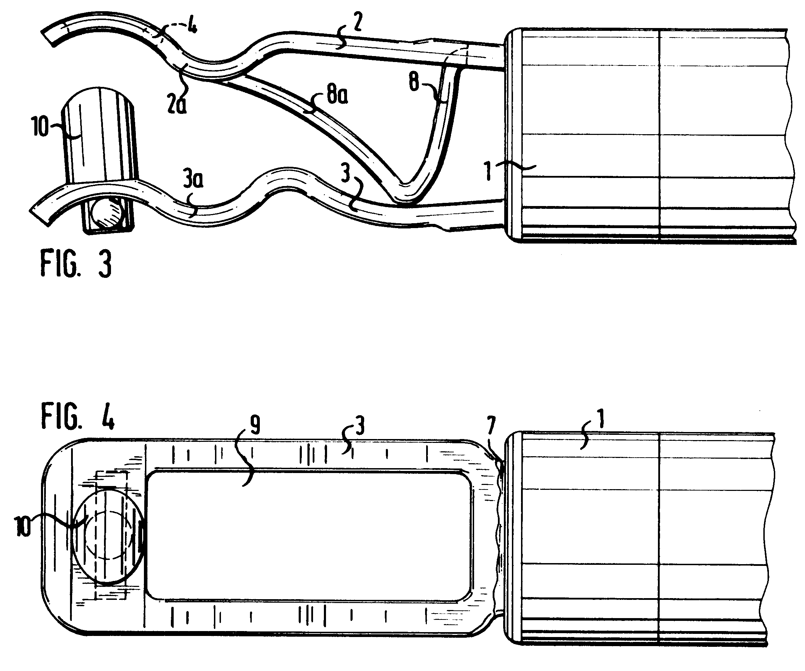

Entsprechend den aus dem zitierten Stand der Technik bekannten Koagulationsfaßzangen sind ein diese bildendes erstes und zweites Maulteil 2 bzw. 3 vorgesehen, die aus dem distalen Ende eines Instrumentenschaftes 1 herausragen. Die Maulteile 2 und 3 sind öffnend durch Federkraft vorgespannt und durch axiale Verschiebung in dem Instrumentenschaft 1 betätigbar, d. h. durch Herausschieben öffenbar und durch Zurückziehen schließbar. Das erste Maulteil 2 bildet in Draufsicht betrachtet (Figur 2) einen Rahmen von im wesentlichen rechteckförmiger Gestalt, der eine Ausnehmung einschließt, die durch einen ersten Quersteg 4 in eine distalwärts gelegene, sich quer zur Instrumentenlängsachse erstreckende erste Ausnehmung 5 von im wesentlichen ovaler Gestalt und eine zweite, in Richtung der Instrumentenlängsachse verlaufende zweite Ausnehmung 6 von in Draufsicht im wesentlichen rechteckiger Gestalt aufgeteilt ist. Dabei wird die zweite Ausnehmung 6 proximal durch einen zweiten Quersteg 7 begrenzt. In Draufsicht betrachtet wird die zweite Ausnehmung 6 in Richtung der Instrumentenachse durch einen Steg 8 symmetrisch unterteilt, der distalseitig an dem ersten Quersteg 4 und proximalseitig an dem zweiten Quersteg 7 fest verankert ist. Der Steg 8 ist wie aus den Figuren 1 und 3 ersichtlich nach unten hakenförmig gekrümmt ausgeführt und greift in Schließstellung der Zange in eine Ausnehmung 9 in dem zweiten Maulteil 3, die im wesentlichen der zweiten Ausnehmung 6 in dem ersten Maulteil 2 entsprechend gestaltet ist. Das zweiten Maulteil 3 ist im Bereich seines distalen Endes mit einem aus PTFE gefertigten Dorn 10 versehen, welcher von dem Schenkel 3 aufragt und an seinem aufragenden Ende atraumatisch ausgebildet ist. Der Dorn 10 taucht in Schließstellung der Zange mit geringem Spiel in die erste Ausnehmung 5 des ersten Maulteils 2 ein. Beide Maulteile 2 und 3 sind wie aus den Figuren 1 und 3 erkennbar wellenförmig gestaltet, so daß sich in Schließstellung (Figur 1) unmittelbar proximalwärts hinter dem Dorn 10 ein bogenförmig eingezogener seitlich offener Bereich 11 zwischen den Maulteilen 2 und 3 ergibt, der durch äquidistante Teilstücke 2a bzw. 3a derselben gebildet wird. In diesem distal durch den Dorn 10 und proximal durch einen Schenkel 8a des Steges 8 begrenzten Bereich 11 wird das zu koagulierende Gewebe aufgenommen.

Die erfindungsgemäße Koagulationszange wird üblicherweise durch eine Trokarhülse in die Körperhöhle eingeführt, wobei sie in geschlossenem Zustand gehalten wird. Nach dem Einführen wird die Koagulationszange geöffnet, so daß der zu koagulierende Eileiter durch die Maulteile 2 und 3 übergriffen werden kann, bis der Eileiter proximalwärts neben dem Dorn 10 und in dem Bereich 11 zwischen den Maulteilen 2 und 3 zu liegen kommt. In dieser Lage befindet sich die Mesosalpinx im Bereich des Dorns 10. Nähern sich nun beim Schließen der Koagulationszange die beiden Maulteile 2 und 3 einander, so wird zunächst die Mesosalpinx druckknopfartig in die erste Ausnehmung 5 hineingestülpt, wobei aufgrund des Spieles zwischen dieser und dem Dorn 10 eine schädigende Scherung vermieden wird. Gleichzeitig dehnt sich der Eileiter zunehmend über den gesamten Bereich 11 aus, wobei dies durch den Schenkel 8a des Steges 8 beim Eintauchen in die Ausnehmung 9 und durch den Dorn 10 begrenzt wird.

Aufgrund der formschlüssigen Fixierung der Mesosalpinx und des Eileiters kann zu dessen Unterbrechung durch Koagulation durch entsprechende Verlagerung der Zange die ganze Gewebestruktur ohne Gefahr einer Beschädigung der Mesosalpinx in die Bauchhöhle gezogen werden, so daß dort ohne Gefährdung umliegenden Gewebes die Koagulation durchgeführt werden kann. Diese konzentriert sich dabei auf den Bereich 11 zwischen den Maulteilen 2 und 3.

## Patentansprüche

1. Bipolare Faßzange zum Koagulieren von Gewebe, mit zwei am distalen Zangenende befindlichen, Elektroden bildenden Maulteilen (2, 3) zum Fassen und Halten des zu koagulierenden Gewebes, wobei an einem Maulteil (2) ein Steg (8) vorgesehen ist, der bei Schließstellung der Maulteile in und ggf. durch das andere Maulteil (3) greift, dadurch gekennzeichnet, daß das eine erste Maulteil (2) am distalen Ende eine Ausnehmung (5) hat und daß am zweiten Maulteil (3) ein Dorn (10) vorgesehen ist, der bei Schließstellung der Maulteile (2, 3) die Ausnehmung (5) mit Spiel durchgreift, wobei der das zu koagulierende Gewebe aufnehmende Bereich (11) zwischen den Maulteilen (2, 3) distal durch den Dorn (10) und proximal durch einen Schenkel (8a) des Steges (8) begrenzt ist.

2. Faßzange nach Anspruch 1, dadurch gekennzeichnet, daß ein Ende des hakenförmig gekrümmten Steges (8) mit einem ersten Quersteg (4) verbunden ist, der die Ausnehmung (5) am distalen Ende des ersten Maulteiles (2) proximal begrenzt, während das andere Stegende mit einem gegenüberliegenden zweiten Quersteg (7) dieses Maulteiles (2) verbunden ist, daß der Steg (8) eine weitere Ausnehmung (6) des ersten Maulteiles (2) überbrückt und in einer Ebene verläuft, die senkrecht auf der Faßebene der Maulteile (2, 3) steht.

3. Faßzange nach einem der Ansprüche 1 und 2, dadurch gekennzeichnet, daß der Steg (8) bei Schließstellung der Maulteile (2, 3) mit dem Scheitel seiner Hakenform in eine Längsausnehmung (9) des zweiten Maulteiles (3) greift und daß der Stegschenkel (8a) vom Scheitel (8b) des Steges (8) bogenförmig zum ersten Quersteg (4) verläuft.

4. Faßzange nach einem der Ansprüche 1 bis 3, dadurch gekennzeichnet, daß die Maulteile (2, 3) auf einem Teil ihrer Länge einen wellenförmigen Verlauf (2a, 3a) aufweisen.

5. Faßzange nach einem der Ansprüche 1 bis 4, dadurch gekennzeichnet, daß der am zweiten Maulteil (3) befestigte, an seinem freien Ende atraumatisch gestaltete Dorn (10) aus PTFE besteht.

## Claims

1. Bipolar gripping forceps for the coagulation of tissue, with two jaw members (2,3) forming electrodes, situated at the distal forceps end, for gripping and holding the tissue which is to be coagulated, wherein on one jaw member (2) a cross-piece (8) is provided, which in the closed position of the jaw members engages in and if necessary through the other jaw member (3), characterised in that the one first jaw member (2) has a recess (5) at the distal end and that on the second jaw member (3) a mandrel (10) is provided which in the closed position of the jaw members (2,3) penetrates the recess (5) with play, wherein the region (11) between the jaw members (2,3)receiving the tissue which is to be coagulated is delimited distally by the mandrel (10) and proximally by a shank (8a) of the cross-piece (8).

2. Gripping forceps according to Claim 1, characterised in that one end of the cross-piece (8), which piece (8) is bent in a hook shape, is connected with a first transverse cross-piece (4), which delimits the recess (5) proximally at the distal end of the first jaw member (2), whilst the other cross-piece end is connected with an opposite, second, transverse cross-piece (7) of this jaw member (2), that the cross-piece (8) bridges a further recess (6) of the first jaw member (2) and extends in a plane which is perpendicular to the gripping plane of the jaw members (2,3).

3. Gripping forceps according to one of Claims 1 and 2, characterised in that the cross-piece (8) in the closed position of the jaw members (2,3) engages with the apex of its hook shape into a longitudinal recess (9) of the second jaw member (3) and that the cross-piece shank (8a) extends from the apex (8b) of the cross-piece (8) in an arc shape to the first transverse cross-piece (4).

4. Gripping forceps according to one of Claims 1 to 3, characterised in that the jaw members (2,3) have a wave-shaped path (2a, 3a) on a part of their length.

5. Gripping forceps according to one of Claims 1 to 4, characterised in that the mandrel (10) which is attached to the second jaw member (3) and is constructed in an atraumatic manner at its free end, consists of PTFE.

## Revendications

1. Pince de préhension bipolaire pour coagulation de tissu, avec deux mors (2, 3) qui se trouvent à l'extrémité distale de la pince et qui forment des électrodes, pour saisir et maintenir le tissu à coaguler, une traverse (8), étant prévue sur un mors (2), traverse qui, en position de fermeture des mors, pénètre dans l'autre mors (3) et, le cas échéant, le traverse, caractérisée en ce que le premier mors (2) a un évidement (5) à l'extrémité distale et qu'un mandrin (10) est prévu sur le second mors (3), mandrin qui, en position de fermeture des mors (2, 3), traverse l'évidement (5) avec du jeu, la zone (11) entre les mors (2, 3), qui recueille les tissus à coaguler, étant limitée distalement par le mandrin (10) et proximalement par un montant (8a) de la traverse (8).

2. Pince de préhension selon la revendication 1, caractérisée en ce qu'une extrémité de la traverse (8) recourbée en forme de crochet est reliée à une première traverse transversale (4) qui limite proximalement l'évidement (5) à l'extrémité distale du premier mors (2), tandis que l'autre extrémité de la traverse est reliée à une seconde traverse transversale (7) opposée de ce mors (2), que la traverse (8) surmonte un autre évidement (6) du premier mors (2) et se situe dans un plan qui est perpendiculaire au plan de préhension des mors (2, 3).

3. Pince de préhension selon l'une des revendications 1 et 2, caractérisée en ce que la traverse (8), en position de fermeture des mors (2, 3), pénètre avec le sommet de sa forme de crochet dans un évidement longitudinal (9) du second mors (3) et que le montant (8a) de la traverse est arqué du sommet (8b) de la traverse (8) vers la première traverse transversale (4).

4. Pince de préhension selon l'une des revendications 1 à 3, caractérisée en ce que les mors (2, 3) ont une allure ondulée (2a, 3a) sur une partie de leur longueur.

5. Pince de préhension selon l'une des revendications 1 à 4, caractérisée en ce que le mandrin (10), qui est fixé sur le second mors (3) et qui est de forme atraumatique à son extrémité libre, est en polytétrafluoréthylène.
